# EUROPEAN PATENT SPECIFICATION

(11) **EP 1 473 015 B1**
(45) Date of publication and mention of the grant of the patent: **15.12.2010**
(21) Application number: 04010114.9
(22) Date of filing: 28.04.2004
(51) Int. Cl.: A61K 6/083

(54) **Dental article and process for surface modification of dental article**
Zahnärtzlicher Artikel und Verfahren für Oberflächenmodifikation des zahnärtzlichen Artikels
Article dentaire et procédé pour la modification de la surface de l'article dentaire

(30) Priority: 28.04.2003 JP 2003124387; 28.10.2003 JP 2003367988
(43) Date of publication of application: 03.11.2004
(73) Proprietor: Tomy Incorporated, Futaba-gun Fukushima-ken (JP)
(72) Inventor: Kubo, Masao, c/o TOMY INCORPORATED, Ohkuma-machi, Futaba-gun, Fukushima-ken (JP); Orikasa, Masaaki, c/o TOMY INCORPORATED, Ohkuma-machi, Futaba-gun, Fukushima-ken (JP)
(74) Representative: Grünecker, Kinkeldey, Stockmair & Schwanhäusser Anwaltssozietät

(56) References cited:
- US-A- 4 579 904
- US-A- 5 889 132
- US-A- 6 057 460
- DATABASE WPI Section Ch, Week 199310 Derwent Publications Ltd., London, GB; Class A96, AN 1993-079486 XP002294780 & JP 05 023353 A (MITSUI PETROCHEM IND CO LTD) 2 February 1993 (1993-02-02)
- PATENT ABSTRACTS OF JAPAN vol. 0172, no. 97 (C-1068), 8 June 1993 (1993-06-08) & JP 5 023354 A (MITSUI PETROCHEM IND LTD), 2 February 1993 (1993-02-02)
- DATABASE WPI Section Ch, Week 200244 Derwent Publications Ltd., London, GB; Class A13, AN 2002-408462 XP002294781 & JP 2001 316432 A (KANEKA CORP) 13 November 2001 (2001-11-13)

## Description

### 1. Field of the Invention

The present invention relates to a dental article to be mounted in the mouth such as orthodontic appliance and denture appliance and a process for a surface modification of a dental article.

### 2. Description of the Related Art

An orthodontic appliance, which is one of dental articles, is used for correcting irregularities of the teeth. Most orthodontic appliances are directly bonded to the teeth of patients for treatment. As such orthodontic appliances, there are known bracket, band, buccal tube, lingual sheath, lingual button, and the like. Among these orthodontic appliances, bracket is a most commonly used appliance.

As an orthodontic appliance, there has heretofore been widely known a metal bracket. In recent years, those formed by transparent or white semi-transparent materials such as ceramic bracket and plastic bracket have been used for the purpose of improving aesthetic appearance.

As the material for forming the current plastic bracket, there is oftenuseda glass fiber-reinforcedpolycarbonate (see, e.g., JP-A-9-98988 and US Patent 5,254,002). Such a polycarbonate bracket is advantageous in that it has a high transparency and a relatively high strength and can be obtained the sufficient bonding strength required for orthodontic treatment with an ordinary orthodontic adhesive.

However, the aforementioned polycarbonate bracket is disadvantageous in that it is discolored by the dye components of foods or drinks to impair its aesthetic appearance, the resin is deteriorated by hydrolysis or the like in an intraoral atmosphere to cause the fracture of the bracket components during orthodontic treatment, it must be replaced within the orthodontic treatment period (normally 18 to 24 months) and bisphenolA (BPA), which is one of endocrine disrupting chemicals (so-called environmental hormone), can be eluted.

The denture appliances such as artificial tooth, denture base and denture back liner, which is one of dental articles, is also formed by various plastics or metals.

As materials for forming, the current denture appliance, there is known a polycarbonate (see, e.g., JP-A-2000-344941), polysulfone or polyphenylsulfone resin, elastomer-containing acrylic resin (see, e.g., JP-A-2001-258908) or polyethylene or polypropylene (see, e.g., JP-A-2002-224144) reinforced by aluminum borate whisker or particulate aluminum borate and the like.

The denture back liner is a device for use in readaptation of ill-adapted denture or treatment of troubles such as pain during mastication. Denture back liners include a hard back liner and a soft back liner. As the materials for forming the hard back liner, there is known an acrylic resin and as the materials for forming the soft back liner, there are known soft acrylic resin and soft silicon resin.

In these denture appliances, bisphenol A can be eluted from denture appliances made of polycarbonate, polysulfone or the like as in the case of the aforementioned polycarbonate bracket. Further, the denture appliance made of acrylic resin have a good moldability but have a disadvantage in that they have a high hygroscopicity and the resin can be easily deteriorated.

Thus, new polymer materials substituting for the aforementioned polycarbonate resin or polyphenylsulfone resin have been desired as materials for forming orthodontic appliances or denture appliances.

For example, orthodonticbrackets formed by polyurethane, polyacetal, acrylic resin or the like have been marketed. However, these brackets are disadvantageous in that they are opaque and certain components canbe eluted. Further, a bracket formed by a transparent nylon (crystallized nylon) has been proposed. However, as the transparent nylon has a high hygroscopicity, such a bracket canbe discolored and deteriorate, and peel off of the teeth surface when exposed to intraoral atmosphere over an extended period of time.

An olefin-based material such as polypropylene and polyethylene is an extremely chemically stable material which undergoes no elution of environmental hormones such as BPA. It is proposed that such a material can be subjected to surface modification to enhance the adhesive property thereof so that it can be used as an orthodontic bracket (see, e.g., JP-A-2002-201297).

On the other hand, the dental articles are occasionally treated with a plastic primer when bonded to the teeth or each other. Aplastic primer is made of a mixture of an acryl monomer, a certain kind of solvent, and the like.

For example, in order to use a primer on a plastic bracket, a plastic primer is applied over the bonding surface of the bracket so that an adhesive can be chemically bonded to the bonding surface of the bracket by dissolving the bonding surface or attaching an adhesive component to the bonding surface. In recent years, however, photopolymerizable adhesives have been applied or preprimed brackets which can be supplied to user with an adhesive applied have been developed. Therefore, such as the primer treatment has been considered ineffective more and more.

As techniques for bonding a bracket to the teeth without treating with a plastic primer, there are known the following ones.

Examples of these techniques include a method which comprises applying an epoxy resin, urethane resin or acrylic resin over the bonding surface of the bracket, sprinkle the coat with particles of silica, alumina or the like while the resin coat is wet, and then hardening the resin coat to form a mechanical lock layer (see, e.g., US Patent 5,267,855), a method which comprises applying a mixture of an acryl monomer and a certain kind of a solvent over the bonding surface of the bracket, and then drying the coat to form a low molecular acryl layer (see, e.g., US Patent 5,295, 824), and a method which comprises applying a mixture of a polymerizable component, a solvent capable of dissolving the bonding surface of the bracket and a photopolymerization initiator over the bonding surface of the bracket, and then drying the coat to form a thin layer (see, e.g., US Patent 5,558,516).

However, the method disclosed in US Patent 5, 267, 855 is disadvantageous in that a definite mechanical lock layer cannot be formed and the other method disclosed in US Patent 5, 295, 824 is disadvantageous in that even the low molecular acryl layer cannot be adapted to the adhesive when it is solidified. Further, the method disclosed in US Patent 5, 558, 516 is disadvantageous in that the thin layer formed on the bonding surface of the bracket undergoes solidification or polymerization with time, making it impossible to obtain a stable bonding strength.

A plastic primer is occasionally used for fixing the artificial teeth to the denture base or repairing a denture.

In this case, solvent crack can occur unless the primer and solvent component are properly selected and used.

An orthodontic bracket made of the olefin-based resin such as polypropylene and polyethylene described in the current art is disadvantageous in that it is not transparent and has a low strength and thus can be easily deformed. When a reinforcing material such as glass fiber, metal and carbon fiber is added to this olefin-based resin to enhance the strength thereof, the transparency of the olefin-based resin is further lost.

The aforementioned problems occurring with plastic brackets occur also with general orthodontic appliances such as band, buccal tube, lingual sheath and lingual button.

Further, denture appliances such as artificial tooth, denture base and denture back liner, if they are formed by an olefin-based resin such as polypropylene and polyethylene, are disadvantageous in that the resin has a low strength and thus can be easily deformed.

JP-A-05023353 relates to an impact absorbing tooth protecting and correcting appliance, made of an ethylene-α-olefin-diene copolymer.

JP-A-05023354 discloses a dental arch protective and orthodontic tool comprising a crosslinked body prepared by crosslinking an ethylene-α-olefin-diene copolymer. The tool has a high-impact absorbency.

US-A-6057460 discloses a polymerizable hybrid monomer having a norbornenyl or norbornadienyl skeleton. The monomer can be readily cured at room temperature and is suitable particular for use in the dental field.

US-A-5889132 relates to a dental material comprising condensation products of silicic acid with norbornene silane or a condensation product of silicic acid with mercapto silane. Product polymerisates have a high mechanical strength.

US-A-4579904 relates to a dental restorative or filling material comprising at least one organic filler, a polymerization initiator, and a polymerization accelerator and a diacrylic or dimethacrylic ester.

### Summary of the Invention

It was the object of the invention to provide a dental article having a high strength which undergoes neither deterioration nor discoloration and providing no elutionof chemical materials such as environmental hormone in an intraoral atmosphere throughout treatment period and shows a high transparency and an aesthetic appearance when used as a orthodontic appliance and further to provide a process for a surface modification of a dental article capable of enhancing an adhesive property of the dental article to the teeth or other dental articles.

Said object is achieved by the dental article of claim 1 and the process for a surface modification of a dental article of claim 3.

Preferred embodiments are set forth in the subclaims.

The term "dental articles" as used herein is meant to indicate orthodontic appliances such as band, bracket, buccal tube, lingual sheath and lingual button, denture appliances such as denture, denture base and denture back liner or other various articles to be mounted in the mouth for dental treatment.

The dental article having the aforementioned arrangement is little subject to deformation because the polymer material obtained by the polymerization of a cycloolefin has a high strength. Further, since the aforementioned polymer material is transparent and chemically stable, the dental article of the invention undergoes neither discoloration nor deterioration in an intraoral atmosphere and causes no elution of materials harmful to human body such as BPA.

The dental article of the invention has a modified bonding surface and is formed by a polymer material obtained by the polymerization of at least a cycloolefin, wherein the surface modification is carried out by applying a graft polymerization initiator over the bonding surface of the article, supplying a reactive monomer onto the coated surface of the article, and then subjecting the coat to graft photopolymerization.

The term "bonding surface of the dental article" as used herein is meant to indicate the bonding surface of the dental article with respect to the teeth or other dental articles.

When the bonding surface of the dental articles is thus subjected to surface treatment, the polymer material at the bonding surface of the dental article can be subjected to grafting, making it possible to enhance the affinity of the polymer material for the adhesive. Accordingly, the dental article thus arranged can have its bonding surface firmly bonded to the adhesive and thus show a sufficient bonding strength when bonded to the teeth or other dental articles.

The dental article of the invention preferably has the bonding surface thereof coated with a photopolymerizable adhesive.

In this arrangement, no troublesome step such as primer coating is required before the bonding of the dental article to the teeth or other dental articles, making it possible to obtain a dental article which can be easily bonded to the object.

On the other hand, the surface modification process of the invention is a process for the surface modification of a dental article formed by a polymer material obtained by the polymerization of at least a cycloolefin, which comprises applying a graft polymerization initiator over the surface of the dental article, supplying a reactive monomer onto the coated surface of the article, and then subjecting the coat to graft photopolymerization.

In accordance with the aforementioned surface modification process, a polymerization initiator is previously applied over the bonding surface of the dental article, making it assured that the surface of the dental article can be subjected to graft polymerization reaction only at required areas. Further, since graft polymerization reaction can be carried out by an easy method such as ultraviolet irradiation, no troublesome steps are required. Accordingly, the surface of the dental article can be more easily and securely modified, making it possible to enhance the adhesive property of the dental article to the teeth or other dental articles.

The dental article of the invention is formed by a transparent polymer material having a high strength obtained by the polymerization of a cycloolefin and thus is little subject to deformation and chemical change such as hydrolysis in an intraoral atmosphere. Accordingly, the invention can provide a dental article having a high strength which undergoes neither deterioration nor discoloration and provides no elution of materials harmful to human body such as BPA. Further, a dental article having a high transparency and an aesthetic appearance can be provided when used as a orthodontic appliance.

Moreover, the process for the surface modification of a dental article of the invention makes it possible to enhance the adhesive property of the dental article to the teeth or other dental articles.

### Brief Description of the Drawings

Fig. 1A is a diagram illustrating a bracket (orthodontic appliance) which is one of the dental articles according to an embodiment of the invention;
Fig. 1B is a diagram illustrating the tooth according to the embodiment of the invention;
Fig. 2A is a schematic diagram illustrating an embodiment of a denture which is one of the dental articles of the invention;
Fig. 2B is a diagram illustrating a denture base; and
Fig. 2C is a diagram illustrating an artificial tooth.

### Detailed Description of the Invention

Embodiments of implementation of the dental article of the invention will be described in detail hereinafter.

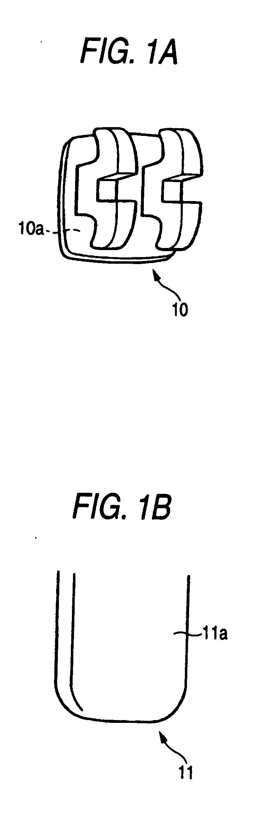

Figs. 1A and 1B are schematic diagrams illustrating an embodiment of a orthodontic appliance which is a dental article of the invention wherein Fig. 1A is a diagram illustrating the bracket and Fig. 1B is a diagram illustrating the teeth.

As shown in Figs. 1A and 1B, a bracket 10 comprises an bonding surface 10a bondable to a surface 11a of teeth 11 and is a formed by a polymer material obtained by the polymerization of at least a cycloolefin.

In the invention, the polymer material constituting dental articles, including orthodontic appliances such as bracket 10, is obtained by the polymerization of at least a cycloolefin (hereinafter referred also to as "cycloolefin-based polymer"). This cycloolefin-based polymer has a high strength and also is very chemically stable.

Examples of the cycloolefin to be used as monomer constituting the cycloolefin-based polymer include cyclopentene, cyclohexene, norbornene, andtetracyclododecene. Preferred among these cycloolefins are norbornene, and tetracyclododecene. These cycloolefins may be used singly or in combination of two or more thereof.

The polymer material according to the invention may be a copolymer of cycloolefin with a monomer other than cycloolefin.

Preferred examples of the form of the polymer material to be used in the invention include product obtained by the polymerization of substantially only the cycloolefin (hereinafter referred also to "polycycloolefin"), and product obtained by the copolymerization of cycloolefin with chain-like olefin.

The dental article formed by such a polycycloolefin is advantageous in that it is excellent in solvent resistance and mechanical strength. Examples of such a polymer include Zeonor 1020R (produced by ZEON CORPORATION).

The dental article formed by the polymer material obtained by the copolymerization of cycloolefin with chain-like olefin is advantageous in that it has a thermoplasticity suitable for molding, a low hygroscopicity, a high chemical resistance to organic solvent, a high rigidity, and the like.

Examples of the chain-like olefin include ethylene, propylene, and 4-methyl-1-pentene. Preferred among these chain-like olefins are ethylene and propylene, and ethylene is particularly preferred. As such a polymer material, there is well known a commercially available product such as Topas 6013 (available from Mitsui Chemicals, Inc.)

The content of repeating units derived from cycloolefin in the entire polymer material obtained by the copolymerization of cycloolefin with chain-like olefin is preferably from 5 to 99% by weight, more preferably from 30 to 99% by weight. The content of repeating units derived from chain-like olefin is preferably from 5 to 70% by weight.

The aforementioned polymer material may be thermoplastic or thermosetting. The polymer material constituting the dental article, if it is thermoplastic, is preferably subjected to injectionmolding, extrusion, blow molding or the like to prepare a dental article. The polymer material which is thermosetting is preferably subjected to injection molding, compression molding or the like to prepare a dental article.

The aforementioned polymer material may comprise glass fiber, metal, carbon fiber, and the like, included therein. In this arrangement, the resulting dental article can be provided with a higher strength. The aforementioned polymer material may contain various additives commonly included or found in polymer materials such as oxidation inhibitor, stabilizer and nucleating agent.

In order to bond the bracket 10 according to the present embodiment of implementation of the invention to the teeth 11, an adhesive to be used in ordinary orthodontic treatment is applied over the entire bonding surface 10a of the bracket 10. The bonding surface 10a is then pressure-bonded to the surface 11a of the teeth 11.

The bracket 10 according to the present embodiment of implementation of the invention as described above is formed by a transparent cycloolefin-based polymer having a high strength. The cycloolefin-based polymer also has a low hygroscopicity and thus is little subject to chemical change such as hydrolysis and provides no elution of materials harmful to human body such as BPA in an intraoral atmosphere. The cycloolefin-based polymer is little subject to discoloration even if it comprises a reinforcing material such as glass fiber, metal and carbon fiber included therein to further enhance the strength thereof. Accordingly, the bracket 10 according to the present embodiment of implementation of the invention is excellent in aesthetic appearance, durability and safety, is little subject to deformation and has a high strength.

The bonding surface 10a of the bracket 10 according to the invention has been subjected to modification by graft photopolymerization according to the invention. In some detail, the surface modification is carried out by applying a graft polymerization initiator over the bonding surface 10a, supplying a reactive monomer onto the coated surface of the bracket, and then irradiating the coated surface of the bracket with light to cause graft photopolymerization. The time at which the surface modification is effect is not specifically limited. However, the surface modification may be effected at any time between shortly after the preparation of the bracket 10 and shortly before the bonding of the bracket 10 to the teeth.

As an embodiment of implementation of the surface treatment process according to the invention, a process for the modification of the bonding surface 10a of the bracket 10 will be described hereinafter.

### (Cleaning)

Firstly, the bonding surface 10a of the bracket 10 is cleaned with, e.g., alcohol, acetone, toluene, singly or in admixture. In an ordinary surface modification of polymer materials, it is normally practiced to clean the surface of the polymer materials.

### (Application of graft polymerization initiator)

Subsequently, a graft polymerization initiator is dissolved in a solvent or the like to prepare a coating solution. The coating solution thus prepared is applied over the cleaned bonding surface 10a, and then dried.

Examples of the graft polymerization initiator herein include benzophenone anthraquinone, peroxides such as benzoyl peroxide, pernitrates such as diammonium cerium nitrate, and persulfates such as ammonium persulfate. As the solvent, there is preferably used one having a high volatility such as acetone and benzene. The concentration of the polymerization initiator in the coating solution is preferably from 0.5 to 10% by weight.

The aforementioned coating solution preferably comprises a hydrophilic polymer such as polyvinyl alcohol and polyvinyl acetate mixed or included therein. In this arrangement, the inhibition of the following graft photopolymerization by oxygen can be prevented. The content of the hydrophilic polymer in the coating solution is preferably from 0.5 to 30% by weight.

### (Execution of graft photopolymerization)

Subsequently, a reactive monomer is applied onto the surface of the bracket 10 coated with the aforementioned polymerization initiator so that graft photopolymerization occurs on the bonding surface 10a. In some detail, the bracket 10 is put in a reaction vessel filled with a solution containing the reactive monomer. The bracket 10 is then irradiated with light.

The wavelength of light with which the bracket is irradiated is not specifically limited, but ultraviolet rays having a wavelength of from 200 nm to 360 nm are preferably used. As the light source, there may be used a high voltage mercury lamp, low voltage mercury lamp, metal halide lamp or the like. During irradiation, a filter may be used to irradiate the bracket with light having a specific wavelength. Alternatively, light having all wavelength ranges emitted by the light source may be used.

The reactive monomer to be used herein is not specifically limited but is preferably a hydrophilic compound having C=C bond (carbon-carbon double bond). Specific examples of such a hydrophilic compound include acrylic acid, methacrylic acid, vinylacetic acid, hydroxyalkyl acrylate, hydroxyalkyl methacrylate, and pyromellitic anhydride. Preferred among these hydrophilic compounds are acrylic acid, and methacrylic acid from the standpoint of bonding strength and chemical resistance.

The aforementioned reactive monomer is preferably dissolved in a solvent which doesn't inhibit the execution of graft photopolymerization such as water, alcohol and mixture thereof. The concentration of the reactive monomer in the solution is preferably from 0.5 to 50% by weight.

### (Post-treatment)

After the termination of the aforementioned graft photopolymerization, the bonding surface of the bracket is then cleaned with a solvent. In this manner, the homopolymer produced by the graft photopolymerization of the hydrophilic polymer with the reactive monomer can be removed with the solvent. By heating the solvent to a temperature of from 50°C to 80°C, the homopolymer can be more easily removed.

Heretofore, methods for the surface modification of articles made of cycloolefin-based polymer have been known in arts other than dental articles. For example, JP-A-2001-316432 and JP-A-2001-318202 disclose a method which comprises heating an optical material comprising a polycycloolefin in a solution of a reactive monomer and a polymerization initiator in a nitrogen atmosphere to cause melt-kneading polymerization or immersion polymerization resulting in grafting.

On the contrary, all the procedures of the graft polymerization in the surface modification method of the invention can be conducted in the atmosphere. Further, grafting can be accomplished by irradiation with ultraviolet rays. Thus, the graft polymerization can be easily effected. Moreover, the application of a polymerization initiator over the bonding surface 10a makes it assured that the reaction can be selectively effectedat the bonding surface 10a. Inthismanner, the surface modification of the bonding surface of the bracket can be easily and certainly effected.

Further, the aforementioned surface modification causes the hydrophilic monomer to undergo graft photopolymerization on the bonding surface 10a, making it possible to render the bonding surface 10a of the bracket 10 more bondable by a hydrophilic chemical material. Accordingly, the affinity of the bonding surface 10a of the bracket 10 for an adhesive can be enhanced, the adhesive canbemore firmly bonded to the bonding surface 10a of the bracket 10 to obtain a sufficient adhesive between the teeth 11 and the bracket 10 when the bracket 10 according to the invention is bonded to the teeth with an adhesive.

Moreover, the bracket 10 according to the present embodiment of implementation of the invention may have a photopolymerizable adhesive applied over the bonding surface thereof. In the case where a photopolymerizable adhesive is applied over the bracket to be subjected to the aforementioned surface modification, the photopolymerizable adhesive is preferably applied after surface modification.

Examples of the photopolymerizable adhesive herein include dental acrylic photopolymerizable adhesives. As these photopolymerizable adhesives, there are well known commercially available products such as Ideal lightcure (available from GAC International Inc. of US) and Reliance LC (available from Reliance Inc. of US).

Further, the bonding surface 10a which has been coated with the photopolymerizable adhesive can then be irradiated with light having a proper wavelength to cause polymerization, making it assured that the photopolymerizable adhesive can be fixed to the bonding surface 10a of the bracket 10.

In order to bond the bracket 10 coated with the photopolymerizable adhesive to the teeth 11, the bonding surface 10a is pressure-bonded to the surface 11a of the teeth 11. The bracket 10 is then irradiated with light having a wavelength of from 250 nm to 500 nm so that the photopolymerizable adhesive is polymerized and fixed to the bonding surface 10a of the bracket 10.

Thus, the bracket 10 further comprising a photopolymerizable adhesive applied over the bonding surface 10a thereof requires no troublesome steps such as primer coating when bonded to the teeth, but merely may be bonded by merely irradiating with light shortly before bonding to the teeth.

While an embodiment of implementation of the dental article and the process for the surface modification thereof according to the invention has been described with reference to orthodontic appliance, the dental article according to the invention is not limited thereto. The invention can be applied to all orthodontic appliances to be used in the correction of irregularities of the teeth such as band, buccal tube, lingual sheath and lingual button or various denture appliances to be mounted in the mouth, including denture appliances such as denture, artificial teeth and denture back liner.

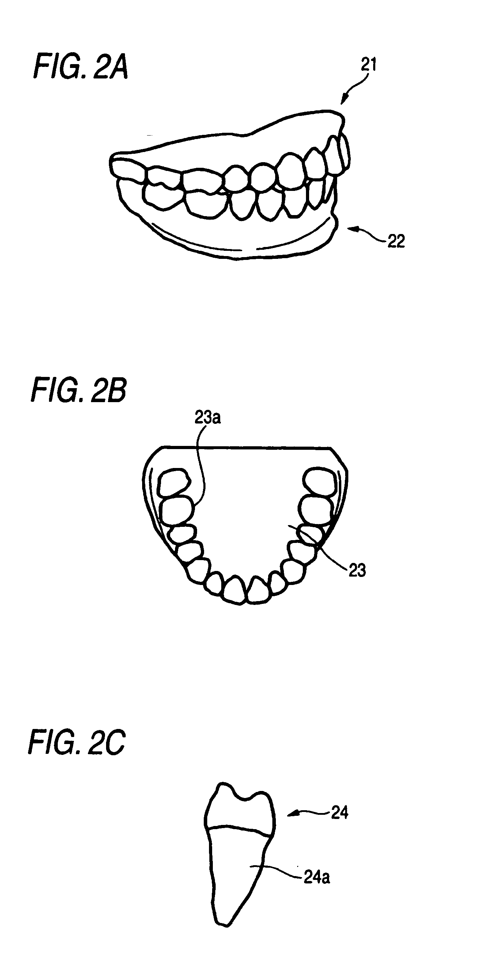

The invention will be further described hereinafter with reference to the case where the invention is applied to denture appliance for use in denture shown in Figs. 2A to 2C. Fig. 2A illustrates a maxillary denture 21 and a mandibular denture 22 each having artificial teeth attached to a denture base. Figs. 2B and 2C each illustrate denture appliances constituting the maxillary denture 21 of Fig. 2A wherein Fig. 2B illustrates a denture base 23 of the maxillary denture 21 and Fig. 2C illustrates an artificial tooth 24.

As shown in Figs. 2B and 2C, the denture base 23 has an bonding surface 23a with respect to the artificial tooth 24 and the artificial tooth 24 has an bonding surface 24a with respect to the denture base 23. The denture base 23 and the artificial tooth 24 each are formed by a cycloolefin-based polymer and can be produced in the same manner as in the procedure of the aforementioned bracket 10 (Figs. 1A and 1B).

Denture articles, such as denture base and artificial tooth, are also formed by a polymer material obtained by the polymerization of at least a cycloolefin and thus have an aesthetic appearance, durability and safety and a high strength and are little subject to deformation. These denture appliances may comprise commonly used additives (e.g., colorant, reinforcing material, stabilizer) properly included therein besides polymer material.

In order to bond the denture base 23 according to the present embodiment to the artificial tooth 24, an adhesive commonly used to bond a denture base to an artificial tooth is applied over the entire bonding surface 23a of the denture base 23 and the entire bonding surface 24a of the artificial tooth 24. The bonding surface 23a and the bonding surface 24a are then pressure-bonded to each other. In the case where the dental article is bonded to other dental articles, it suffices if at least one of the dental articles is formed by a cycloolefin-based polymer. However, both the two dental articles are preferably formed by a cycloolefin-based polymer.

In this case, the bonding surface 23a of the denture base 23 and/or the bonding surface 24a of the artificial tooth 24 is also subjected to modification by the graft photopolymerization of the invention as in the case of the aforementioned bracket 10. The bonding surface 23a of the denture base 23 and/or the bonding surface 24a of the artificial tooth 24 may be subjected to modification of the invention in the same manner as in the case of the aforementioned bracket 10. When the bonding surfaces 23a and 24a are thus subjected to modification of the invention, the adhesive property of the denture base 23 and the artificial tooth 24 can be enhanced.

Further, the denture base 23 and artificial tooth 24 according to the present embodiment of implementation of the invention may have a photopolymerizable adhesive applied over the bonding surfaces 23a and 24a thereof, respectively. In order to applied a photopolymerizable adhesive over the denture base 23 and the artificial tooth 24 to be subjected to the aforementioned surface modification, it is preferred that the photopolymerizable adhesive be applied after- the aforementioned surface modification. Moreover, as the photopolymerizable adhesive, there may be used one described above. The application of the photopolymerizable adhesive can be effected in the same manner as in the case of the bracket 10.

Thus, the denture base 23 and artificial tooth 24 comprising a photopolymerizable adhesive applied over the bonding surface 23a and 24a thereof require no troublesome steps such as primer coating when bonded to the object but merely may be irradiated with light shortly before bonding to the object.

### [Example]

The invention will be further described in the following examples and comparative examples, but the invention should not be construed as being limited thereto.

### [Example 1]

### (Preparation of bracket)

A polycycloolefin (trade name: Zeonor 1020R; glass transition temperature: 105°C; produced by ZEON CORPORATION) was subj ected to ordinary injection molding to prepare a bracket 10 as shown in Fig. 1A. The sum of weights of ten brackets 10 thus obtained was 0.265 g.

### (Surface modification of bracket)

The bracket thus prepared was cleaned with ethanol at room temperature for 30 minutes, and then dried at 50°C for 30 minutes.

Subsequently, an acetone solution containing 3% by weight of a polyvinyl acetate as a hydrophilic polymer and 2% by weight of benzophenone as a graft polymerization initiator was applied over the bonding surface of the bracket, and then dried at 50°C for 30 minutes. Thereafter, 100 ml of an aqueous solution containing 5% by weight of acrylic acid as a reactive monomer was put in a reaction vessel made of quartz glass. The bracket was then put in the reaction vessel. The bracket was then irradiated with ultraviolet rays from a Type Toscure HC-411A 400W high voltage mercury lamp (produced by Toshiba Corporation) disposed at a distance of 20 cm from the liquid level for 60 minutes. The temperature of the solution thus irradiated was 50°C.

The bracket thus irradiated with ultraviolet rays was cleaned with hot water having a temperature of from 60°C to 70°C for 30 minutes, cleaned with acetone for 5 minutes, and then dried at 80°C for 30 minutes.

In this manner, "Bracket A", which had been subjected to graft photopolymerization on the bonding surface with respect to the surface of the tooth, was obtained. The rise of the weight of the bracket after the aforementioned treatment was 0.2%.

### [Example 2]

The procedure of Example 1 was followed except that a copolymer of cycloolefin with ethylene (Topas 6013; thermal deformation temperature: 130°C; available from Mitsui Chemicals, Inc.) was used instead of polycycloolefin. The bracket thus obtained was then subjected to surface modification on the bonding surface thereof in the same manner as in Example 1 to obtain "Bracket B".

### [Example 3]

A bracket was prepared in the same manner as in Example 1. The bracket thus obtained was then subjected to surface modification on the bonding surface thereof in the same manner as in Example 1. The bracket thus prepared was coated with a Bis-GMA-based photopolymerizable adhesive (trade name: Ideal lightcure, available from GAC International Inc. of US) on the bonding surface thereof under illumination having components of 600 nm or less cut off by filtration. The bracket thus coated was then stored in a black sealed vessel for 7 days to obtain "Bracket C".

### [Illustrative Example 4]

The procedure of Example 1 was followed except that the bonding surface of the bracket was not subjected to modification to prepare "Bracket D".

### [Example 5]

### (Preparation of denture base)

A polycycloolefin (trade name: Zeonor 1020R; glass transition temperature : 105°C; produced by ZEON CORPORATION) was subj ected to ordinary in j ection molding to prepare a denture base 23 as shown in Fig. 2B. The weight of the denture base 23 thus obtained was 13.25 g.

### (Surface modification of denture base)

The denture base thus prepared was cleaned with ethanol at room temperature for 30 minutes, and then dried at 50°C for 30 minutes.

Subsequently, an acetone solution containing 3% by weight of a polyvinyl acetate as a hydrophilic polymer and 2% by weight of benzophenone as a graft polymerization initiator was applied over the bonding surface of the denture base with respect to artificial tooth, and then dried at 50°C for 30 minutes. Thereafter, 200ml of an aqueous solution containing 5% by weight of acrylic acid as a reactive monomer was put in a reaction vessel made of quartz glass. The denture base was then put in the reaction vessel. The denture base was then irradiated with ultraviolet rays from a Type Toscure HC-411A 400W high voltage mercury lamp (produced by Toshiba Corporation) disposed at a distance of 20 cm from the liquid level for 60 minutes. The temperature of the solution thus irradiated was 45°C.

The denture base thus irradiated with ultraviolet rays was cleaned with hot water having a temperature of from 60°C to 70°C for 30 minutes, cleaned with acetone for 5 minutes, and then dried at 80°C for 30 minutes.

In this manner, "Denture base A", which had been subjected to graft photopolymerization on the bonding surface with respect to the surface of artificial tooth, was obtained. The rise of the weight of the denture base after the aforementioned treatment was 0.12%.

### [Example 6]

The procedure of Example 5 was followed except that a copolymer of cycloolefin with ethylene (Topas 6013; thermal deformation temperature: 130°C; available from Mitsui Chemicals, Inc.) was used instead of polycycloolefin. The denture base thus obtained was then subjected to surface modification on the bonding surface thereof with respect to artificial tooth in the same manner as in Example 5 to obtain "Denture base B".

### [Example 7]

A denture base was prepared in the same manner as in Example 5. The denture base thus obtained was then subj ected to surface modification on the bonding surface thereof with respect to artificial tooth in the same manner as in Example 5. The denture base thus prepared was coated with a Bis-GMA-based photopolymerizable adhesive (trade name: Ideal lightcure, available from GAC International Inc. of US) on the bonding surface thereof with respect to artificial tooth under illumination having components of 600 nm or less cut off by filtration. The denture base thus coated was then stored in a black sealed vessel for 7 days to obtain "Denture base C".

### [Illustrative Example 8]

The procedure of Example 5 was followed except that the bonding surface of the denture base with respect to artificial tooth was not subjected to modification to prepare "Denture base D".

### [Comparative Example 1]

The procedure of Example 1 was followed except that a polypropylene (trade name: J-754HP, produced by Idemitsu Petrochemical Co., Ltd.) was used instead of polycycloolefin. The bracket thus obtained was then subjected to surface modification to obtain "Bracket E".

### [Comparative Example 2]

The polypropylene used in Comparative Example 1 was used to prepare "Bracket F". However, the surface modification effected in Comparative Example 1 was not effected.

### [Comparative Example 3]

The procedure of Comparative Example 1 was followed except that a glass fiber-reinforced polypropylene (trade name: L-3040P; glass fiber content: 30%; produced by Idemitsu Petrochemical Co., Ltd.) was used instead of polypropylene. The bracket thus obtained was then subjected to surface modification in the same manner as in Comparative Example 1 to obtain "Bracket G".

### [Comparative Example 4]

The glass fiber-reinforced polypropylene used in Comparative Example 3 was used to prepare "Bracket H". The modification of the bonding surface of the bracket effected in Comparative Example 3 was not effected.

### [Comparative Example 5]

The procedure of Comparative Example 1 was followed except that a polycarbonate (trade name: Lexan, produced by GE Plastics Inc.) was used instead of polypropylene to prepare "Bracket I". However, the surface modification effected in Comparative Example 1 was not effected.

### [Comparative Example 6]

The procedure of Example 5 was followed except that a polyethylene (trade name: 0628G, produced by Idemitsu Petrochemical Co., Ltd.) was used instead of cycloolefin. The denture base thus prepared was then subjected to surface modification in the same manner as in Example 5 to obtain "Denture base E".

### [Comparative Example 7]

The polyethylene used in Comparative Example 6 was used to prepare "Denture base F". However, the surface modification effected in Comparative Example 6 was not effected.

### [Comparative Example 8]

The procedure of Comparative Example 6 was followed except that a mixture of a polymethyl methacrylate (tradename: ACRYCON, produced by Mitsubishi Rayon Co., Ltd.) and 0.5% by weight of benzoyl peroxide was used instead of polyethylene to prepare "Denture base G". However, the surface modification effected in Comparative Example 6 was not effected.

### [Comparative Example 9]

The procedure of Comparative Example 6 was followed except that a polycarbonate (trade name: Lexan, produced by GE Plastics Inc.) was used instead of polyethylene to prepare "Polycarbonate-made denture base H". However, the surface modificationeffected in Comparative Example 6 was not effected.

### [Evaluation]

### (Light transmittance)

Aspecimen having a thickness of 3 mmwas cut out of Brackets A to I thus prepared. These specimens were each then tested for light transmittance according to ASTM D1003.

### (Tensile break test)

Brackets A to I and Denture bases A to H thus prepared were each fixed to a Type 5567 tensile testing machine (produced by Instron Ltd. of US). The tensile strength required when the deformation in the tensile direction reaches 10% was then measured.

### (Colorability test)

Three dyes, i.e., Dianix Blue AC-E, Dianix Yellow AC-E and Dianix Red AC-E (all produced by Dyestar Japan, Ltd.) were each dissolved in water in a concentration of 3% by weight. The solution was then heated to a temperature of from 70°C to 80°C

In the solution were then each put Brackets A to I and DenturebasesAtoH. The solution was then heated for 30 minutes. Thereafter, these brackets and denture bases were each boiled and cleaned in water containing a neutral detergent, and then dried. These products were then compared for colorability.

The colorability was evaluated according to the following criterion.
E: Little colored
P: Colored

### (Shear adhesive strength test)

Brackets A, B and D and Comparative Brackets E to I thus prepared were each coated with an adhesive on the bonding surface thereof, and then bonded to an adherend which is a model of the tooth (alumina ceramic in the form of cylinder having a diameter of 8 mm and a height of 10 mm which is silane-treated on the bonding surface thereof with respect to bracket). As the adhesive, there was used a Bis-GMA-based adhesive (trade name: Accubond; two-paste type, chemically-polymerized type; available from GAC International Inc. of US). The adherend to which the bracket had been bonded was allowed to stand at 37°C in the atmosphere for 24 hours, and then measured for shear adhesive strength using a Type 5567 tensile testing machine (produced by Instron Ltd. of US).

Bracket C was pressure-bonded and fixed to the same adherend as used above on the bonding surface thereof, and then irradiated with light having a wavelength around 470 nm (600 to 750 mW/cm²) for 40 seconds to cause the polymerization of the adhesive. Thereafter, the adherend to which the bracket had been bonded was allowed to stand at 37°C in the atmosphere for 24 hours, and then measured for shear adhesive strength using a Type 5567 tensile testing machine (produced by Instron Ltd. of US).

Denture bases A, B and D and Comparative denture bases E to H thus prepared were each coated with an adhesive on the bonding surface thereof with respect to artificial tooth (denture mounting surface). An artificial tooth (trade name: Surpass (anterior tooth) C3, produced by GC Dental Co., Ltd.) was then bonded to these denture bases. As the adhesive, there was used a Bis-GMA-based adhesive (trade name: Accubond; two-paste type, chemically-polymerized type; available from GAC International Inc. of US).

The denture base to which the artificial tooth had been bonded were each allowed to stand at 37°C in the atmosphere for 24 hours, and then measured for shear adhesive strength using a Type 5567 tensile testing machine (produced by Instron Ltd. of US).

A denture was bonded to Denture base C in the same manner as mentioned above. Denture base C was then irradiated with light having a wavelength around 470 nm (600 to 750 mW/cm²) for 80 seconds to cause the polymerization of the adhesive. Thereafter, the denture base to which the artificial tooth had been bonded was allowed to stand at 37°C in the atmosphere for 4 hours, and then measured for shear adhesive strength using a Type 4456 tensile testing machine (produced by Instron Ltd. of US).

The results are set forth in Table 1.

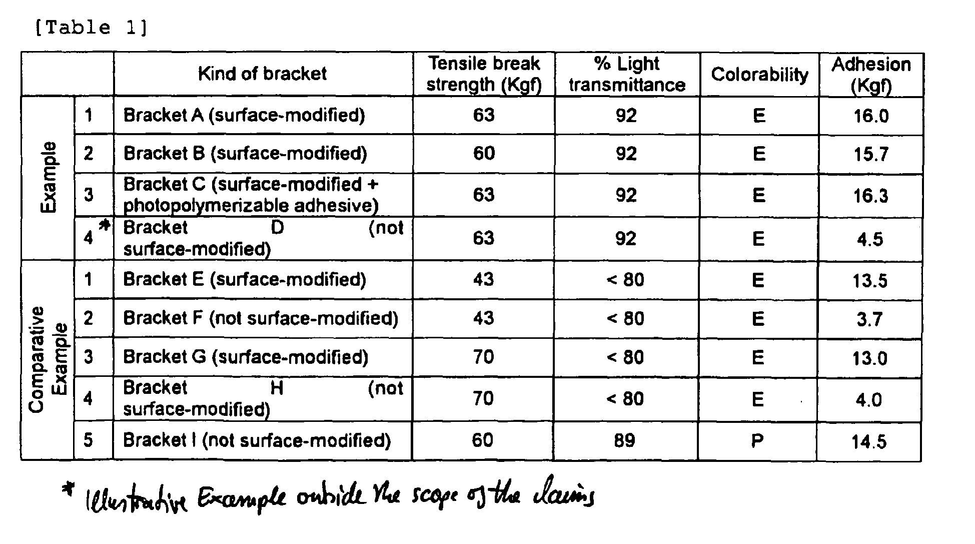

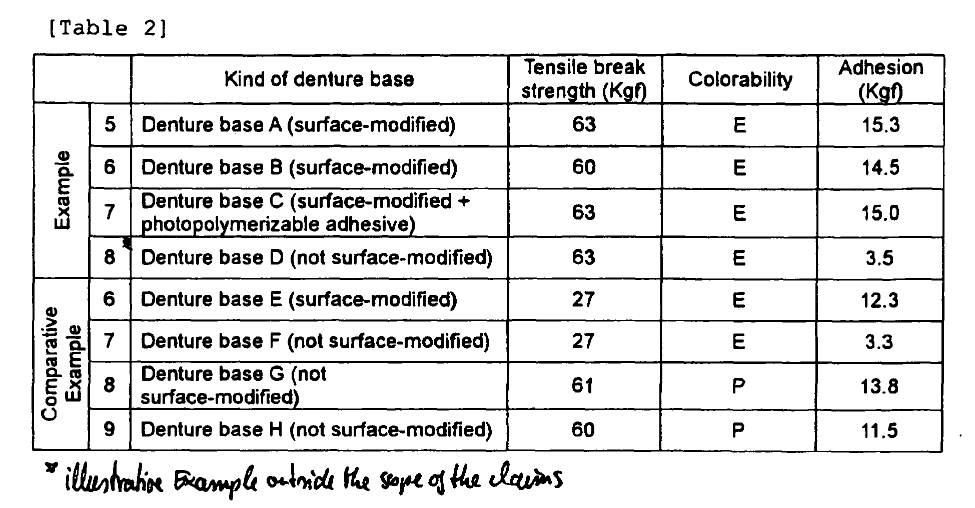

As can be seen in the results of Table 1, the cycloolefin-based brackets A to C of the invention are excellent in tensile break strength, light transmittance and colorability as compared with the comparative brackets E to I. As can be seen in the results of Table 2, the denture bases A to C of the invention are excellent in tensile break strength and colorability as compared with the comparative denture bases.

The surface modification of the invention makes it possible to enhance the bonding strength of these articles. It can also be seen that the surface modification of the invention can remarkably enhance the adhesive strength of the bracket and denture base of the invention in particular.

Bracket D (not surface-modified) is inferior to Bracket I (not surface-modified), which is made of a polycarbonate, in adhesion. However, Bracket I shows an extremely poor colorability and thus cannot be used as a orthodontic appliance. Similarly, Denture base D (not surface-modified) is inferior to Denture bases G and H (both not surface-modified) in adhesion. However, Denture bases G and H show an extremely poor colorability and thus cannot be used as a denture appliance.

## Claims

1. A dental article having a modified bonding surface formed by a polymer material obtained by a polymerization of at least a cycloolefin, wherein a surface modification is carried out by applying a graft polymerization initiator over the bonding surface of the dental article, supplying a reactive monomer onto the coated surface of the article, and then subjecting the coat to graft photopolymerization.

2. The dental article as defined in claim 1, wherein the bonding surface comprises a photopolymerizable adhesive applied thereover.

3. A process for a surface modification of a dental article formed by a polymer material obtained by a polymerization of at least a cycloolefin, said process comprising;
applying a graft polymerization initiator over a surface of the dental article,
supplying a reactive monomer onto the coated surface of the article; and
subjecting the coat to graft photopolymerization.

## Patentansprüche

1. Dentalartikel, der eine modifizierte Klebeoberfläche aufweist, die durch ein Polymermaterial gebildet wird, das durch eine Polymerisation wenigstens eines Cycloolefins gewonnen wird, wobei eine Oberflächenmodifikation ausgeführt wird, indem ein Pfropf-Polymerisationserreger auf die Klebeoberfläche des Dentalartikels aufgetragen wird, der beschichteten Oberfläche des Artikels ein reaktives Monomer zugeführt wird und die Beschichtung dann Pfropf-Photopolymerisation unterzogen wird.

2. Dentalartikel nach Anspruch 1, wobei die Klebeoberfläche einen fotopolymerisierbaren Klebstoff umfasst, der auf sie aufgetragen ist.

3. Prozess für eine Oberflächenmodifikation eines Dentalartikels, der durch ein Polymermaterial gebildet wird, das durch eine Polymerisation wenigstens eines Cycloolefins gewonnen wird, wobei der Prozess umfasst:
Auftragen eines Pfropf-Polymerisationserregers auf eine Oberfläche des Dentalartikels;
Zuführen eines reaktiven Monomers zu der beschichteten Oberfläche des Artikels; und
Durchführen von Pfropf-Photopolymerisation der Beschichtung.

## Revendications

1. Article dentaire présentant une surface de liaison modifiée formée par un matériau polymère obtenu par une polymérisation d'au moins une cyclooléfine, dans lequel une modification de surface est réalisée en appliquant un initiateur de polymérisation de greffage sur la surface de liaison de l'article dentaire, en fournissant un monomère réactif sur la surface revêtue de l'article, et en soumettant ensuite le revêtement à une photopolymérisation de greffage.

2. Article dentaire selon la revendication 1, dans lequel la surface de liaison comprend un adhésif photopolymérisable appliqué sur son dessus.

3. Procédé pour une modification de surface d'un article dentaire formée par un matériau polymère obtenu par une polymérisation d'au moins une cyclooléfine, ledit procédé comprenant les étapes consistant :
à appliquer un initiateur de polymérisation de greffage sur une surface de l'article dentaire ;
à fournir un monomère réactif sur la surface revêtue de l'article ; et
à soumettre le revêtement à une photopolymérisation de greffage.
